# EUROPEAN PATENT SPECIFICATION

(11) **EP 0 971 765 B1**
(45) Date of publication and mention of the grant of the patent: **10.07.2002**
(21) Application number: 97947988.8
(22) Date of filing: 24.11.1997
(51) Int. Cl.: A61M 16/04

(54) **ARTIFICIAL RESPIRATION DEVICE**
KÜNSTLICHES BEATMUNGSGERÄT
DISPOSITIF DE RESPIRATION ARTIFICIELLE

(30) Priority: 28.11.1996 NL 1004640
(43) Date of publication of application: 19.01.2000
(73) Proprietor: Ideamed N.V., Willemstad, Curacao (AN)
(72) Inventor: VAN DEN BERG, Paulus, Cornelis, Maria, NL-1103 PR Amsterdam (NL)
(74) Representative: de Bruijn, Leendert C.
(86) International application number: PCT/NL97/00641
(87) International publication number: WO 98/23317

(56) References cited:
- EP-A- 0 284 335
- WO-A-92/13587
- WO-A-95/06492
- NL-A- 6 615 648
- US-A- 2 541 691
- US-A- 4 840 172
- US-A- 5 514 153

## Description

The present invention relates to an artificial respiration device for a human or mammal, comprising a tube having a distal end which is intended to be guided, via the mouth and the pharynx, in the direction of the trachea, and a proximal end which is intended to be disposed outside of the humans oral cavity, an inflatable sealing ring being arranged at said distal end of the tube, wherein the inflatable sealing ring is intended for forming a seal between a wall of said tube and a wall of the humans pharynx, wherein the sealing ring is positioned on the tube asymmetrically with respect to the axis of the tube in the position in which the distal end has been introduced into the pharynx and inflated.

Such an artificial respiration device is disclosed in the international patent application WO 95/06492.

The artificial respiration devices employed according to the prior art can be devided into two categories, an endotracheal tube and larynx mask. An endotracheal tube is introduced, via the oral cavity and the pharynx, as far as into the trachea, past the vocal chords. A balloon is attached to the thin flexible tube, close to and around the distal end thereof. After placing the tube in position, the balloon is inflated, so that the space between the outer wall of the tube and the walls of the trachea is closed off in an airtight manner. Then, artificial respiration means can be used to build up air pressure in the trachea and in the lungs, which are at a lower level.

Endotracheal tubes have the significant advantage that their operational reliability is extremely high, and hence so is their safety in use. For this reason, the tubes are also employed when a patient has to be artificially respirated for a long period of time. Another advantage is that the tubes are inexpensive to manufacture and therefore can be used as disposable items. However, the fact that the distal end of the tube, with a balloon attached to it, has to be placed between the vocal chords is a major drawback of endotracheal tubes. The vocal chords serve to protect the trachea and they have to be loosened in order to allow the artificial respiration tube to be introduced. Moreover, the vocal chords are very delicate. When the artificial respiration tube is being introduced, the vocal chords are touched and damage may be caused to the vocal chords. This may lead to infections, the formation of scar tissue, the trachea becoming blocked or to permanent hoarseness for the patient.

Introducing the endotracheal tube into the trachea also entails the risk of numerous complications, such as colonization, tracheobronchitis and pneumonia.

An additional drawback of the use of an endotracheal tube is that the cross-section of the tube is considerably narrower than the cross-section of the trachea itself. This leads to a considerable increase in the resistance to airflow between the mouth and the trachea. Since artificial respiration tubes are often used for people who have problems breathing, this is a serious shortcoming.

Relatively expensive larynx masks are characterized by the fact that they run, via the oral cavity and the pharynx, as far as into the entry to the trachea, but do not pass the larynx. The end of a larynx mask is provided with a specifically shaped, complicated, inflatable body, which is able to close off the pharynx above the trachea in an airtight manner. The inflatable body is attached in such a way that it bears against the chest side of the pharynx. After inflating the inflatable body, the larynx mask is connected in an airtight manner to the entry to the trachea. A major advantage of larynx masks is that their use does not pose any risk to the vocal chords, since the masks are not inserted into the trachea.

However, a number of drawbacks are associated with the use of larynx masks. The use of these masks does not guarantee the same degree of safety as the use of endotracheal tubes. If a patient moves during artificial respiration, the risk of leakage is high.

Moreover, the inflatable body of a larynx mask has to be placed under high pressure in order to obtain the airtight closure. This pressure is so high that larynx masks can never be used for long periods of time, in order to prevent damage to the layer of mucus in the pharynx. Also, larynx masks provide less protection for the trachea against the penetration of, for example, liquid from the oesophagus than endotracheal tubes.

An additional drawback of the use of larynx masks is that they cannot be used as disposable articles, owing to their high cost price. After using a mask, it is not thrown away but rather sterilized. The use of larynx masks thus requires the presence of sterilization means.

International patent application WO-92/13587 has disclosed an artificial respiration device which attempts to combine the advantages of the use of a larynx mask with the advantages of the use of an endotracheal tube. The device essentially comprises an outer tube (10), a larynx mask (12) being attached to the end thereof and an endotracheal tube (40), with an inflatable sealing ring at one end, being inserted through the said outer tube. However, a number of drawbacks are associated with the use of the artificial respiration device according to this international patent application; the larynx mask according to the abovementioned patent application is relatively large. The reason for this is that most of the mask comprises a solid, non-inflatable member. For this reason, among others, the mask cannot be introduced by visual control with the aid of a laryngoscope, but rather has to be manoeuvred blind, by touch, into the correct position. With patients who are unable to open their mouth wide, the larynx mask according to this application may even not pass through the teeth. Since the closure of the oesophagus has to be ensured by the larynx mask, there is just as much risk of aspiration when using a device according to the abovementioned application as when using conventional larynx masks. In this case, the distal tube opening in the larynx mask is pushed backwards when the inflatable ring (14) is inflated, which in fact increase the risk of aspiration further.

Since the closure of the larynx is also carried out with the aid of the cuff (12), when using the abovementioned mask the sealing-ring pressure has to be very high, in order to be able to achieve an airtight closure. Hence the use of this artificial respiration device according to the abovementioned application entails the same risks with regard to damaging the layer of mucus in the pharynx as the use of conventional larynx masks.

An additional drawback of the larynx mask according to WO-92/13587 is that the closure achieved is not very effective, despite the high pressure in the mask. If patients are to be artificially respirated, the artificial-respiration pressure which is necessary to fully inflate the lungs often causes leakage past the mask. This leakage may cause the ventilation of the lungs to fall considerably, which may lead to serious complications.

Furthermore, the form of the larynx mask according to WO-92/13587 means that it is only possible to ensure that it is fixed in the correct position by taping the tube to the skin around the outside of the mouth, with the aid of plasters, for example. The form of the larynx mask even makes it possible for the larynx mask to slip out of position and to pass from the hypopharynx into the oropharynx and the oral cavity. This then endangers the most important function which it was desired to achieve by means of the larynx mask, namely a safe, freely accessible airway.

Another artificial respiration device of the type mentioned in the introduction is described in international patent application WO-95/06492. This patent application describes an artificial respiration device which comprises, inter alia, a tube (12) and an inflatable sealing ring (14). It is clear from the text and the drawings of this international patent application that the oropharynx (that part of the pharynx which is behind the tongue) is closed with the aid of the sealing ring (14). The aim of the artificial respiration device according to this international patent application is to close off the nasopharynx by raising the palatum molle. Moreover, the airway is closed off at the level of the oropharynx. By means of the inflated sealing ring (14), the base of the tongue is lifted, and consequently the epiglottis is lifted indirectly. Moreover, the sealing ring (14) is provided, in the inflated state, with a projection (28) on the front of the sealing ring (14), in order to hold the artificial respiration device behind the position of the tongue. An important aim of the artificial respiration device according to WO-95/06492 is to prevent manipulation by the larynx. This can only be achieved by inserting the artificial respiration tube no further than into the oropharynx. In order to operate correctly, the tube may not be placed in the hypopharynx.

The artificial respiration device according to this patent application has a number of significant limitations as a result of the design selected. Firstly, access to the oesophagus is not closed off. There is therefore open communication between the lungs and the stomach via the hypopharynx. This means that during the artificial respiration the contents of the stomach can pass into the lungs. This is a serious limitation, since aspiration of the contents of the stomach into the lungs can lead to serious pneumonia.

According to the text of this patent application, the artificial respiration device functions only when breathing spontaneously or under artificial respiration if low pressures are used. For this reason, the artificial respiration devices according to this patent application can only be used under anaesthetic for short interventions.

The object of the present invention is to provide an artificial respiration device which combines the advantages of using endotracheal tubes and larynx masks, without also adopting the drawbacks thereof. In particular, the invention aims to provide an artificial respiration device which exhibits a good closure, and thus little risk of leakage, prevents the contents of the stomach from passing into the lungs in the event of vomiting, cannot cause damage to the larynx and is inexpensive and hence disposable. This object is achieved in the present invention in that the tube has a length for introducing the distal end into the entry to the trachea, for positioning the sealing ring in the hypopharynx, and that in the position in which the sealing ring has been introduced in the hypopharynx and inflated the sealing ring is mainly disposed adjacent to one side of said distal end of the tube and has a shape and dimensions for essentially filling the hypopharynx and for pressing the tube towards the entry to the trachea.

The advantage of an artificial respiration device according to the present invention is that the inflatable sealing ring will essentially be situated between the tube and the rear side of the pharynx. This means that when the sealing ring is inflated the distal end of the tube will press away from the rear side of the pharynx, in the direction of the opening of the trachea. As a result, firstly the distal end of the tube is positioned in front of the opening to the trachea, and secondly the distal end of the tube is prevented from being able to touch and/or damage the delicate attachment of the vocal chords at the tapering separation between the trachea and the oesophagus.

Since the artificial respiration device according to the present invention is not inserted into the trachea, but runs as far as the entry to the trachea, the internal diameter of the artificial respiration tube can be selected to be approximately just as large as the external diameter of endotracheal tubes according to the prior art. This has the advantage that the resistance to the flow of air through the tube is relatively low compared to the resistance to the flow of air from endotracheal tubes.

In order to be able to close off the space above the entry to the trachea in an airtight manner, it is advantageous if the inflatable sealing ring, in the inflated state, is adapted to the anatomy of the pharynx. The pharynx has its smallest cross-section between the back of the tongue and the rear wall of the pharynx. The largest cross-section of the throat is at the level of the entry to the oesophagus and the trachea. In the inflated state, the sealing ring has to fill up this shape. This is achieved by the fact that the size of the cross-sectional area of a sealing ring perpendicular to the axis of the tube increases in the direction towards the distal end of the tube, and moreover by the fact that the sealing ring, in the longitudinal direction along the axis of the tube, essentially has the shape of a trapezium, with a relatively small top side facing away from the distal end of the tube, and a relatively large bottom side facing towards the distal end of the tube.

The advantage of the shape of the sealing ring according to the present invention is, inter alia, that this shape ensures that an airtight closure of the pharynx is maintained, even if the patient starts to move or vomit during the artificial respiration. The shape of the sealing ring means that the tube will not move further into the body when the sealing ring is being inflated. Also, the tube is not pressed out of the body by the sealing ring. Any further movement of the tube is minimal if the mouth of a patient is closed off from above with the aid of tape. When using the present invention, it is advisable to tape up the mouth, in the same way as is usual when using other artificial respiration devices.

Moreover, it is advantageous if the shape of the sealing ring prevents any build-up of liquid in the oral cavity from entering the trachea. This is achieved by the fact that the said relatively small top side of the sealing ring forms an angle α of less than 90° with the axis of the tube. This has the advantage that any build-up of liquid presses the top side of the sealing ring more firmly against the wall. In this way, the sealing ring prevents liquid from moving past the sealing ring, towards the entry to the trachea.

It is attempted to prevent any build-up of liquid at the relatively large bottom side from being able to pass into the trachea. This is achieved by the fact that the said relatively large bottom side forms an angle β of less than 90° with the axis of the tube, and by the fact that the outer wall of the annular sealing ring runs at an angle γ with respect to the axis of the tube in such a manner that the outer wall moves away from the tube in the direction towards the distal end of the tube.

The advantage of this form is that any build-up of liquid on the relatively large bottom side can be conveyed towards the oral cavity, along the rear side of a sealing ring arranged in the throat.

The artificial respiration device can be improved if a stop is used in the vicinity of the entry to the trachea when the tube is being positioned. This is achieved by the fact that a strip, which is intended to be introduced into the top end of the oesophagus, is attached to the tube, close to the distal end of the latter which is to be guided towards the trachea. Preferably, the said strip is L-shaped and, in the out-of-use position of the artificial respiration device, forms a slight angle (10-20°) with the axis of the tube, which angle, when the tube has been introduced into the larynx and the sealing ring has been inflated, is reduced to approximately 0°.

When the tube is being introduced, the strip will slide into the oesophagus. The strip thus guides the distal end of the tube towards the trachea. The strip presents the distal end of the tube from being pushed into the trachea. The strip allows the tube to be introduced and the distal end of the tube, which faces towards the trachea, to be positioned without the need for any other accessories and without extensive training.

Moreover, the use of the strip also provides significant protection against accumulated liquid or the contents of the stomach penetrating into the trachea.

For reasons of safety, preferably it is not just the sealing ring which is used to close off the area on the lung side of the sealing ring in an airtight manner. This is achieved by the fact that a sealing member is arranged at the distal end of the tube, in order to seal the tube against the walls of the trachea at the entry to the latter. The advantage of a sealing member of this kind, which already largely closes off the entry to the trachea in an airtight manner, is that the pressure in the sealing ring can be kept at a relatively low level without there being any risk of leakage taking place past the sealing ring. One design of a sealing member could comprise a ring of mutually overlapping, protruding, flexible flaps.

From the US patent 5.514.153 an active canula or sleeve is known which is usuable to create and/or enlarge in channel or passage to position a scope or instrument or to move or locate tissue. According to the American patent the canula can comprise a bladder retractor, fixed to the canula. The plurality of circumferential extending reinforcing fibers are embedded in an elastomeric matrix material. Upon introduction of fluid under pressure through the canula, the bladders expand longitudinally and define a rigid structural unit. Using a canula or sleeve or artificial respiration of a human or mammal is not disclosed in this US patent.

From the Dutch patent application 6.615.648 an artificial respiration device is known, comprising a flexible, extendable umbrella-formed extending part. In this document using a ring of nutury overlapping, protuding, flexible flaps, is not disclosed.

Preferably, the said sealing member can be introduced into the trachea in the closed state. This can be achieved by means of a cap which, when the sealing member is being introduced, surrounds the flexible flaps and is connected to a thread which leads to the start of the tube. The advantage of this is that the entry to the trachea is not damaged during introduction of the sealing member. In order to be able to introduce extra air or oxygen, the said thread may be hollow.

The operation and use of the artificial respiration tube according to the present invention is explained with reference to the following figures, which show an exemplary embodiment.

Figure 1 shows a side view of the artificial respiration device according to the present invention, in the out-of-use position.

Figure 1a shows a side view of a detail of the artificial respiration device according to the present invention, in the in-use position.

Figure 2 shows a front view of the artificial respiration device according to the present invention.

Figure 3 shows a sketch of the artificial respiration device according to the present invention in the inserted position.

Figure 4 shows a side view of the end part of the device, on an enlarged scale.

A sealing ring 2 is arranged on an artificial respiration tube 1, close to the distal end thereof, i.e. that part which is directed towards the trachea. In the drawing, the sealing ring 2 is attached to the rear side, in the deployed position, of the tube. The cross-section of the sealing ring 2 shown in the figure, perpendicular to the axis of the tube, is essentially in the shape of a kidney. It should be noted that the sealing ring 2 may also surround the tube completely, in the form of a ring. In the deployed position, in both cases most of the volume of the sealing ring 2 is situated on the rear side of the tube.

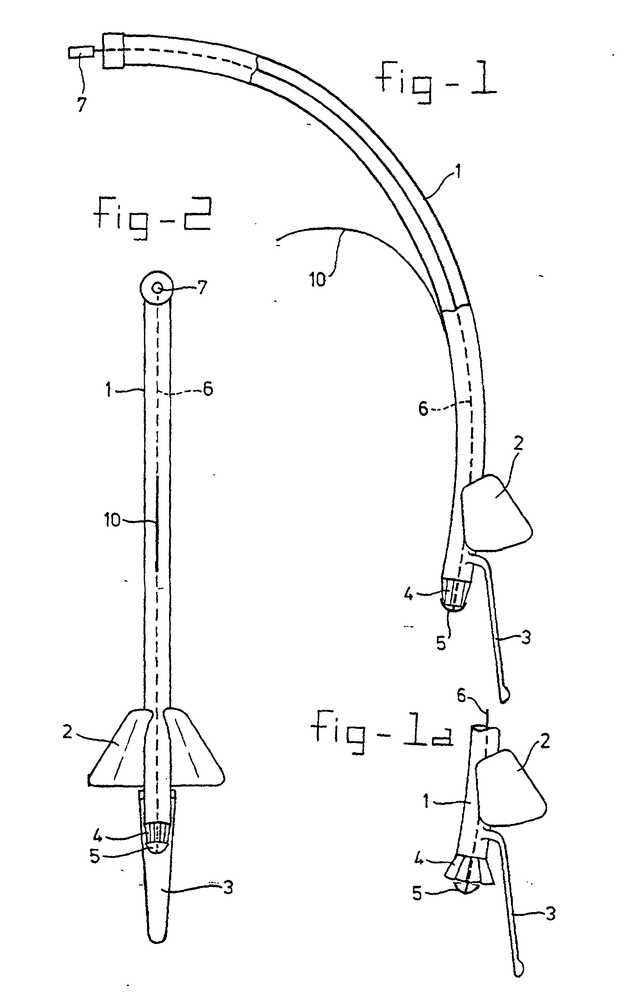

It can be seen in Figures 1 and 2 that the strip 3 narrows. The lower part of the strip 3 is rounded, which provides the advantage that the strip 3 cannot damage the layer of mucus in the oesophagus. After the tube 1 has been introduced, the strip 3 closes off the connection between oesophagus and trachea. This provides considerable protection to the trachea and the lungs, in the event that the patient starts to vomit during artificial respiration.

A sealing member 4 is attached to the distal end of the tube 1. The sealing member 4 serves to close off the opening of the trachea. In the embodiment shown, the sealing member 4 comprises a number of separate flaps, for example made of silicone rubber, which partially overlap one another. The flaps each have a length of about 10 mm. As shown in Figure 1, a cap 5 holds the sealing member 4 together. In Figure 1a, it can be seen that the cap 5 can be moved further towards the distal end of the tube 1, with the aid of a thread 6, so that the cap 5 is removed from the sealing member 4 and the flaps can fold apart, coming to lie at an angle of approximately 45° with respect to the axis of the tube 1.

The thread 6 is made of a hard synthetic material or of a soft synthetic material with a core made of metal. Since the thread is stiff and has a certain curvature, the thread is able to facilitate the introduction of the tube 1. The thread 6 can be actuated with the aid of a knob 7 at the start of the tube 1.

The sealing ring 2 can be inflated by means of a channel 10 which is partially accommodated in the wall of the tube (1).

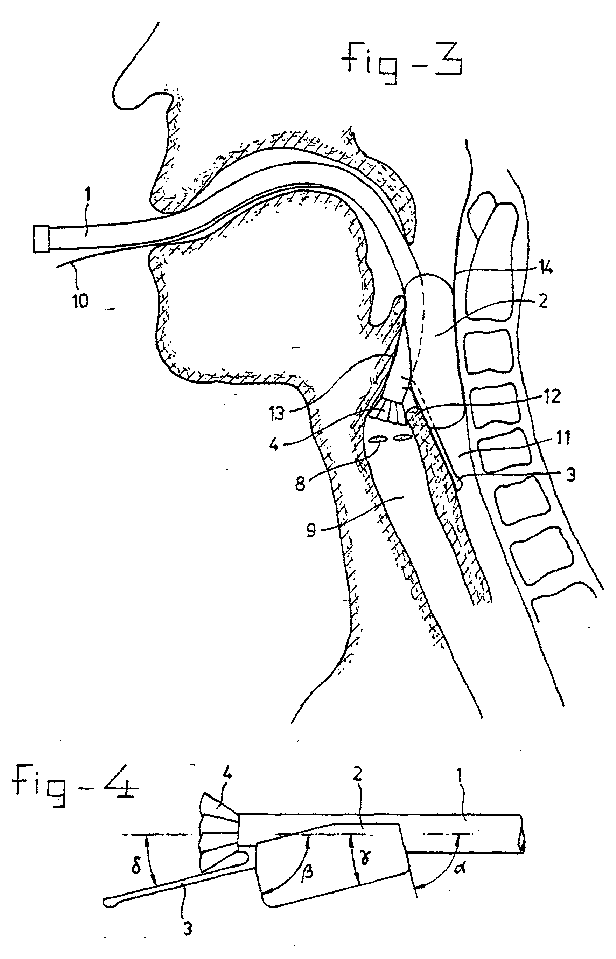

Figure 3 shows the tube 1, as inserted into the pharynx of a person via the mouth. The sealing ring 2, which is attached at the distal end of the tube 1, is inflated and its external curvature bears against the rear side of the pharynx 14. In so doing, the sealing ring 2 presses the distal end of the tube 1 towards the opening of the trachea 9. Moreover, the sealing ring 2 presses the tube 1 against the chest side of the pharynx 13. The strip 3 bears against the tapered partition 12 between the trachea 9 and the oesophagus 11. The sealing ring 2 and the strip 3 position the distal end of the tube in such a way that the sealing member 4 rests in the trachea 9, above the vocal cords 8. Hence the strip 3 provides extra protection against the penetration of liquid from the oesophagus 11 into the opening of the trachea 9. The presence of the strip 3 means that minimum training is required in order to introduce the tube into the trachea 9 at the position which is desired for the vocal cords 8. Moreover, the strip ensures that the tube cannot be pushed into the oesophagus 11. When the tube 1 is being put in place, the strip 3 forms a certain angle (10°-20°) with the axis of the tube 1. When the sealing ring is inflated, this angle will decrease to approximately 0°.

It can be seen in Figure 3 that the shape of the sealing ring 2 is matched to the anatomy of the pharynx. That side of the sealing ring 2 which faces towards the chest closes off the access from the oral cavity to the trachea 9. Moreover, the bottom side of the sealing ring 2 closes off the access to the oesophagus 11. As can clearly be seen from the drawing, the connection between the trachea 9 and the oesophagus is also closed off. As a result, the risk of aspiration is limited to a minimum when using the device according to the present invention. In the inflated state, the position of the tube is essentially fixed. During inflation of the sealing ring 2, the tube 1 will no longer enter further into the body, and will also not be forced out of the body by the sealing ring 2. Moreover, the risk of leakage past the sealing ring if the patient moves or turns or if the patient moves his/her neck or head, is low, due to the shape of the sealing ring 2. This is particularly true if the mouth of the patient has been taped shut.

Since the tube cannot be inserted into the trachea, but only moves as far as the entry to the trachea, the internal diameter of the tube 1 may be selected to be just as large as the external diameter of endotracheal tubes according to the prior art. This means that the resistance which the flow of air undergoes in the tube is less than if an endotracheal tube were to be used.

Pressure can be built up in the trachea 9 beneath the sealing member 4. The sealing member 4 and the sealing ring 2 then prevent air from leaking out of the trachea in the direction towards the pharynx and the oral cavity.

As can be seen from Figure 4, the relatively small top side of the sealing ring 2, which side faces away from the distal end of the tube, forms an angle α of less than 90° with the axis of the tube. The bottom side, facing towards the distal end of the tube, of the sealing ring 2, or at least that part of this side which faces towards the rear, forms an angle β of less than 90° with the axis of the tube. The outer wall of the sealing ring 2 forms an angle γ with the axis of the tube which is such that the outer wall moves further away from the tube, when seen in the direction towards the distal end of the tube.

An L-shaped strip 3 is attached close to the distal end of the tube, which strip is pushed into the oesophagus when the tube is being inserted and helps to position the distal end of the tube. The strip guides the tube in such a manner that it comes to rest centrally in front of the opening of the airway.

An additional advantage is that the artificial respiration device can be used as a disposable item, owing to its simple design and its price.

The design of the artificial respiration device shown in the figures and discussed is given only by way of example.

## Claims

1. Artificial respiration device for a human or mammal, comprising a tube (1) having a distal end which is intended to be guided, via the mouth and the pharynx, in the direction of the trachea, and a proximal end which is intended to be disposed outside of the humans oral cavity, an inflatable sealing ring (2) being arranged at said distal end of the tube, wherein the inflatable sealing ring (2) is intended for forming a seal between a wall of said tube (1) and a wall of the humans pharynx, wherein the sealing ring (2) is positioned on the tube (1) asymmetrically with respect to the axis of the tube (1) in the position in which the distal end has been introduced into the pharynx and inflated, **characterized in that**, the tube has a length for introducing the distal end into the entry to the trachea, for positioning the sealing ring (2) in the hypopharynx, and that in the position in which the sealing ring (2) has been introduced in the hypopharynx and inflated the sealing ring (2) is mainly disposed adjacent to one side of said distal end of the tube (1) and has a shape and dimensions for essentially filling the hypopharynx and for pressing the tube towards the entry to the trachea.

2. Artificial respiration device according to Claim 1, **characterized in that** the size of the cross-sectional area of the sealing ring (2) perpendicular to the axis of the tube (1), in the inflated state, increases in the direction towards the distal end of the tube.

3. Artificial respiration device according to Claim 2, **characterized in that** the sealing ring (2), in the inflated state, in longitudinal section along the axis of the tube (1), essentially has the shape of a trapezium, with a relatively small top side facing away from the distal end of the tube, and a relatively large bottom side facing towards the distal end of the tube.

4. Artificial respiration device according to Claim 3, **characterized in that** the said relatively small top side of the sealing ring (2) forms an angle α of less than 90° with the axis of the tube (1).

5. Artificial respiration device according to Claim 3, **characterized in that** the said relatively large bottom side forms an angle β of less than 90° with the axis of the tube (1).

6. Artificial respiration device according to Claim 3, 4 or 5, **characterized in that** the outer wall of the sealing ring (2) runs at an angle γ with respect to the axis of the tube (1) in such a manner that the outer wall moves away from the tube (1) in the direction towards the distal end of the tube.

7. Artificial respiration device according to one of the preceding claims,
**characterized in that** a strip (3), which is intended to be introduced into the top end of the oesophagus, is attached to the tube (1), close to the distal end of the latter which is to be guided towards the trachea.

8. Artificial respiration device according to Claim 7, **characterized in that** the said strip (3) is L-shaped and, in the out-of-use position of the artificial respiration device, forms a slight angle (10-20°) with the axis of the tube (1), which angle, when the tube has been introduced into the larynx and the sealing ring (2) has been inflated, is reduced to approximately 0°.

9. Artificial respiration device according to one of the preceding claims, **characterized in that** a sealing member (4) is arranged at the distal end of the tube (1), in order to seal the tube (1) against the walls of the trachea (9) at the entry to the latter.

10. Artificial respiration device according to Claim 9, **characterized in that** the said sealing member (4) comprises a ring of mutually overlapping, protruding, flexible flaps.

11. Artificial respiration device according to Claim 10, **characterized by** a cap (5) which, when the sealing member (3) is being introduced, surrounds the flexible flaps (4) and is connected to a thread (6) which leads to the start of the tube (1).

12. Artificial respiration device according to Claim 10, **characterized in that** the thread (6) is hollow.

## Patentansprüche

1. Künstliches Beatmungsgerät für Menschen oder Säugetiere mit:
einem Rohr (1) mit einem distalen Ende, das dazu vorgesehen ist, über den Mund und den Rachen in Richtung der Luftröhre geführt zu werden, und einem proximalen Ende, das dazu vorgesehen ist, außerhalb der Mundhöhle angeordnet zu werden;
einem am distalen Ende des Rohrs angeordneten, aufblasbaren Dichtungsring (2), wobei der aufblasbare Dichtungsring (2) dazu vorgesehen ist, eine Dichtung zwischen einer Wand des Rohrs (1) und der Wand des Rachens zu erzeugen, wobei der Dichtungsring (2) bezüglich der Achse des Rohrs (1) in der Position, in der das distale Ende in den Rachen eingeführt ist, im aufgeblasenen Zustand asymmetrisch auf dem Rohr (1) angeordnet ist;
**dadurch gekennzeichnet, daß**
das Rohr eine Länge aufweist, die es ermöglicht, das distale Endes in den Eingang der Luftröhre einzuführen, um den Dichtungsring (2) im Hypopharynx zu positionieren, und daß in der Position, in der der Dichtungsring (2) in den Hypopharynx eingeführt und aufgeblasen worden ist, der Dichtungsring (2) im wesentlichen in der Nähe einer Seite des distalen Endes des Rohrs (1) angeordnet ist und eine Form und Abmessungen aufweist, um den Hypopharynx im wesentlichen auszufüllen und das Rohr zum Eingang der Luftröhre hin zu drücken.

2. Künstliches Beatmungsgerät nach Anspruch 1, **dadurch gekennzeichnet, daß** die Größe der Querschnittsfläche des Dichtungsrings (2) senkrecht zur Achse des Rohrs (1) im aufgeblasenen Zustand in Richtung zum distalen Ende des Rohrs hin zunimmt.

3. Künstliches Beatmungsgerät nach Anspruch 2, **dadurch gekennzeichnet, daß** der Dichtungsring (2) im aufgeblasenen Zustand im Längsschnitt entlang der Achse des Rohrs (1) im wesentlichen die Form eines Trapezes aufweist, wobei eine relativ kleine Oberseite vom distalen Ende des Rohrs abgewandt ist und eine relativ große Unterseite dem distalen Ende des Rohrs zugewandt ist.

4. Künstliches Beatmungsgerät nach Anspruch 3, **dadurch gekennzeichnet, daß** die relativ kleine Oberseite des Dichtungsrings (2) einen Winkel α von weniger als 90° bezüglich der Achse des Rohrs (1) bildet.

5. Künstliches Beatmungsgerät nach Anspruch 3, **dadurch gekennzeichnet, daß** die relativ große Unterseite einen Winkel β von weniger als 90° bezüglich der Achse des Rohrs (1) bildet.

6. Künstliches Beatmungsgerät nach Anspruch 3, 4 oder 5, **dadurch gekennzeichnet, daß** die Außenwand des Dichtungsrings (2) unter einem Winkel γ bezüglich der Achse des Rohrs (1) derart verläuft, daß die Außenwand sich in Richtung zum distalen Ende des Rohrs hin vom Rohr (1) weg bewegt.

7. Künstliches Beatmungsgerät nach einem der vorangehenden Ansprüche, **dadurch gekennzeichnet, daß** ein Streifen (3), der dazu vorgesehen ist, in das obere Ende der Speiseröhre eingeführt zu werden, in der Nähe des distalen Endes des Rohrs (1), das dazu vorgesehen ist, zur Luftröhre hin geführt zu werden, am Rohr befestigt ist.

8. Künstliches Beatmungsgerät nach Anspruch 7, **dadurch gekennzeichnet, daß** der Streifen (3) L-förmig ist und, wenn das künstliche Beatmungsgerät nicht in Gebrauch ist, einen kleinen Winkel (10-20°) bezüglich der Achse des Rohrs (1) bildet, wobei der Winkel, wenn das Rohr in den Larynx eingeführt und der Dichtungsring (2) aufgeblasen worden ist, auf etwa 0° abnimmt.

9. Künstliches,Beatmungsgerät nach einem der vorangehenden Ansprüche, **dadurch gekennzeichnet, daß** ein Dichtungselement (4) am distalen Ende des Rohrs (1) angeordnet ist, um das Rohr (1) gegen die Wände der Luftröhre (9) an ihrem Eingang zu dichten.

10. Künstliches Beatmungsgerät nach Anspruch 9, **dadurch gekennzeichnet, daß** das Dichtungselement (4) einen Ring aus wechselseitig überlappenden, hervorstehenden, flexiblen Lappen oder Laschen aufweist.

11. Künstliches Beatmungsgerät nach Anspruch 10, **gekennzeichnet durch** eine Abdeckung (5), die, wenn das Dichtungselement (3) eingeführt wird, die flexiblen Lappen (4) umschließt und mit einem Faden (6) verbunden ist, der zum Anfang des Rohrs (1) führt.

12. Künstliches Beatmungsgerät nach Anspruch 10, **dadurch gekennzeichnet, daß** der Faden (6) hohl ist.

## Revendications

1. Dispositif de respiration artificielle destiné à un être humain ou à un mammifère, comprenant un tube (1) comportant une extrémité distale qui est prévue pour être guidée, par l'intermédiaire de la bouche et du pharynx, dans la direction de la trachée, et une extrémité proximale qui est prévue pour être disposée à l'extérieur de la cavité buccale des êtres humains, un anneau d'étanchéité gonflable (2) étant disposé au niveau de ladite extrémité distale du tube, dans lequel l'anneau d'étanchéité gonflable (2) est prévu en vue de former un joint d'étanchéité entre une paroi dudit tube (1) et une paroi du pharynx des êtres humains, dans lequel l'anneau d'étanchéité (2) est positionné sur le tube (1) de façon asymétrique par rapport à l'axe du tube (1) dans une position dans laquelle l'extrémité distale a été introduite dans le pharynx et gonflée, **caractérisé en ce que** le tube présente une longueur en vue d'une introduction de l'extrémité distale dans l'entrée de la trachée, afin de positionner l'anneau d'étanchéité (2) dans l'hypopharynx, et **en ce que** dans la position dans laquelle l'anneau d'étanchéité (2) a été introduit dans l'hypopharynx et gonflé, l'anneau d'étanchéité (2) est disposé principalement de façon contiguë à un côté de ladite extrémité distale du tube (1) et présente une forme et des dimensions en vue de remplir essentiellement l'hypopharynx et en vue de presser le tube en direction de l'entrée vers la trachée.

2. Dispositif de respiration artificielle selon la revendication 1, **caractérisé en ce que** la taille de la surface de section transversale de l'anneau d'étanchéité (2) perpendiculaire à l'axe du tube (1), à l'état gonflé, augmente dans la direction vers l'extrémité distale du tube.

3. Dispositif de respiration artificielle selon la revendication 2, **caractérisé en ce que** l'anneau d'étanchéité (2), à l'état gonflé, en coupe longitudinale suivant l'axe du tube (1), présente essentiellement la forme d'un trapèze irrégulier, un côté supérieur relativement petit regardant dans le sens opposé à l'extrémité distale du tube, et un côté inférieur relativement grand regardant vers l'extrémité distale du tube.

4. Dispositif de respiration artificielle selon la revendication 3, **caractérisé en ce que** ledit côté supérieur relativement petit de l'anneau d'étanchéité (2) forme un angle α de moins de 90° avec l'axe du tube (1).

5. Dispositif de respiration artificielle selon la revendication 3, **caractérisé en ce que** ledit côté inférieur relativement grand forme un angle β de moins de 90° avec l'axe du tube (1).

6. Dispositif de respiration artificielle selon la revendication 3, 4 ou 5, **caractérisé en ce que** la paroi extérieure de l'anneau d'étanchéité (2) s'étend à un angle γ par rapport à l'axe du tube (1) de telle manière que la paroi extérieure s'écarte du tube (1) en direction de l'extrémité distale du tube.

7. Dispositif de respiration artificielle selon l'une des revendications précédentes, **caractérisé en ce qu'**une bande (3), qui est prévue pour être introduite dans l'extrémité supérieure de l'oesophage, est fixée au tube (1), près de l'extrémité distale de ce dernier qui doit être guidée vers la trachée.

8. Dispositif de respiration artificielle selon la revendication 7, **caractérisé en ce que** ladite bande (3) est en forme de L et, dans la position hors utilisation du dispositif de respiration artificielle, forme un léger angle (10 à 20°) avec l'axe du tube (1), lequel angle, lorsque le tube a été introduit dans le larynx et que l'anneau d'étanchéité (2) a été gonflé, est réduit à approximativement 0°.

9. Dispositif de respiration artificielle selon l'une des revendications précédentes, **caractérisé en ce qu'**un élément d'étanchéité (4) est disposé au niveau de l'extrémité distale du tube (1), de manière à appuyer hermétiquement le tube (1) contre les parois de la trachée (9) au niveau de l'entrée vers cette dernière.

10. Dispositif de respiration artificielle selon la revendication 9, **caractérisé en ce que** ledit élément d'étanchéité (4) comprend un anneau de lamelles souples, faisant saillie, se chevauchant mutuellement.

11. Dispositif de respiration artificielle selon la revendication 10, **caractérisé par** une coiffe (5) qui, lorsque l'élément d'étanchéité (3) est introduit, entoure les lamelles souples (4) et est reliée à un fil (6) menant au début du tube (1).

12. Dispositif de respiration artificielle selon la revendication 10, **caractérisé en ce que** le fil (6) est creux.
